# EUROPEAN PATENT APPLICATION

(11) **EP 4 640 249 A1**
(43) Date of publication of application: **29.10.2025**
(21) Application number: 24020131.9
(22) Date of filing: 26.04.2024
(51) Int. Cl.: A61M 1/00

(54) **SELF-PRIMING BLADDER FLUSH PUMP (PBFP)**

(71) Applicant: Swarts, Johan J.H., 9545 TZ Bourtange (NL)
(72) Inventor: Swarts, Johan J.H., 9545 TZ Bourtange (NL)

(57) **Abstract**

Self-priming electric blow irrigation device with on the right a input unit, for connecting a flushing tube and catheter. On the left an output unit, for connecting the drain flushing hose for a catheter bag, or the hose can be placed in another reservoir, for example the sink. Through a moment switch (SPST) the pump is switched on and starts sucking when it is pressed and held. As soon as it is released, the pump stops sucking. Because an inlet connecting tube is connected to a urinary catheter with double lateral eyes, slightly rotated to each other, measuring 7 × 4.5 mm, many debris, stones and impurities in the bladder can be effectively removed/drained. The patient or helper can clearly hear that the pump makes more noise if the bladder wall closes one of the lateral eyes. The patient, helper, or nurse then releases the switch and can pull the catheter slightly back and press the switch again.

The device is intended to ensure smooth urine drainage and prevent infection in patients. This can be done with a partially filled bladder of at least 100ml of urine and/or by first injecting/squeezing a bag of bladder irrigation fluid into the bladder through the catheter. You can also use a 2-way or 3-way catheter to permanently connect a bag of bladder irrigation fluid, and then simultaneously have the pump suck and flush fluid run into the bladder. The device gives a huge boost to bladder management, which gives the possibility to preventively remove bladder stones, prevent clumping of stones, and thus no longer provide a source for infections.

## Description

The invention relates to a Self-Priming electric Flush Pump_device, abbreviated: the PBFP. Comprising a plastic splash-proof base/housing, in which an electric DC pump is mounted. On the right is an input unit, for connecting a flushing hose and a catheter (preferably: the Rusch 221800200 - Rusch Siliconized Tiemann Intermittent Catheter, 20 Ch. Which gave very good results) and on the left an output unit, for connecting the drain flushing hose on a catheter bag, or the hose can be placed in another reservoir, for example a sink. A small moment switch (SPST) on the front of the housing, ensures that the pump turns on and starts sucking when pressed and held. As soon as it is released, the pump stops sucking. Because an inlet connecting tube is connected to a urinary catheter with double, slightly rotated in relation to each other lateral eyes of 7 x 4.5 mm, many grit, stones and impurities in the bladder can be effectively removed/drained. The pump will not suck the bladder wall vacuum because of the double lateral eyes. The patient can clearly hear that the pump makes more noise if the bladder wall sucks on one of the eyes, or a stone closes one of the eyes. The patient, helper, or nurse then releases the switch and can pull the catheter slightly back and press the switch again. This works in the same way as regular catheterization to empty the bladder as well as possible. (If necessary, the device can be equipped with an LED indicator that lights up, if the pump has a too heavy load because the pump sucks on the bladder wall and seals one of the lateral eyes, or if there is no more fluid/urine flowing). Also the pump can be equipped with a automatically turn off/stop, if the load is too heavy.

The device is intended to ensure smooth urine drainage and prevent infection in patients with long-term, or permanent, impaired bladder function. This can be done with a partially filled bladder of at least 100ml of urine (preferably more) and/or by first injecting/squeezing a bag of bladder irrigation fluid into the bladder though the catheter. You can also permanently connect a bag of bladder irrigation fluid by means of a 2-way or 3-way catheter, or by connecting tap water with a capacity of up to 25 ml/second to the tap and simultaneously let the device sucked. In this way, the bladder is flushed even more effectively of impurities. The patient is no longer dependent on only the "capillary action" and the (reducing) muscle contraction either of the bladder itself, or through the siphon principle.

The pump has a maximum capacity of 25 ml/second and a maximum displacement of 50 cm, in order to be able to effectively suck up all stone dust through the lateral eyes. Uroflowmetry: In general, peak flow rates (Qmax) of 15-20 mL/second are considered normal for adult men. The device gives a huge boost to bladder management, which gives the possibility to preventively remove bladder stones, prevent clumping of stones, and thus no longer provide a source for infections.

The utility model describes a device that can also be used mobile and at home, but also in hospitals and/or care homes. It is for daily use on bladder stones already present, or for periodic maintenance of the bladder to prevent stone formation that accumulates in the bladder and causes bladder spasms and bladder infection. In this way, the formation of bladder stones is nipped in the bud. This results in fewer urinary tract infections and hospital surgeries, where the stones have to be crushed.

The workload of medical staff and costs are significantly reduced.

To achieve the above-mentioned invention goal, the utility model uses the following technical solutions: DC pump 12V 4.4A and a capacity of 1.5L/minute (25 ml/second). By means of a small momentary switch (SPST) the pump can be activate. A power supply (adapter) can be connected through a Low-voltage connector, Bus construction type, installation with inner Ø 6.3mm. As an option, a rechargeable lithium battery could also be placed in the housing.

By adopting the above-mentioned technical solution, the utility model has the following beneficial effects: maintenance of the bladder to prevent stone formation, fewer urinary tract infections, fewer bladder cramps. The utility model discloses a quantitative and effective blow flushing and emptying device, with a simple structure and easy to use.

### SHORT DESCRIPTION OF THE DRAWINGS

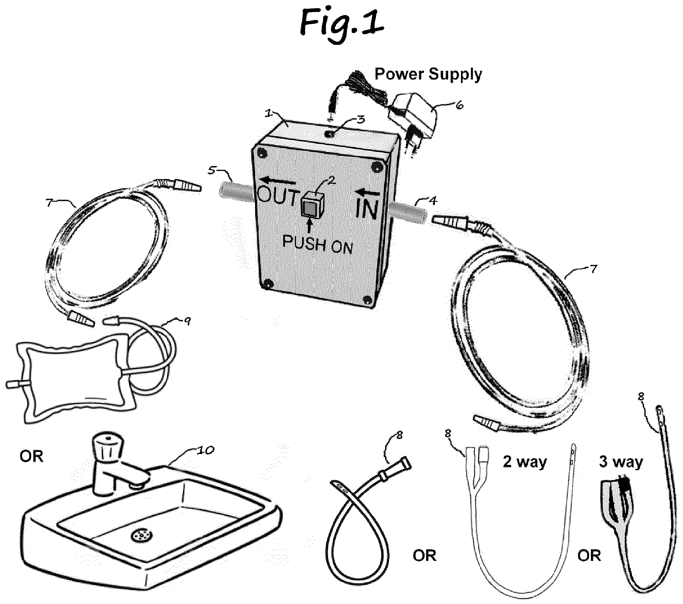

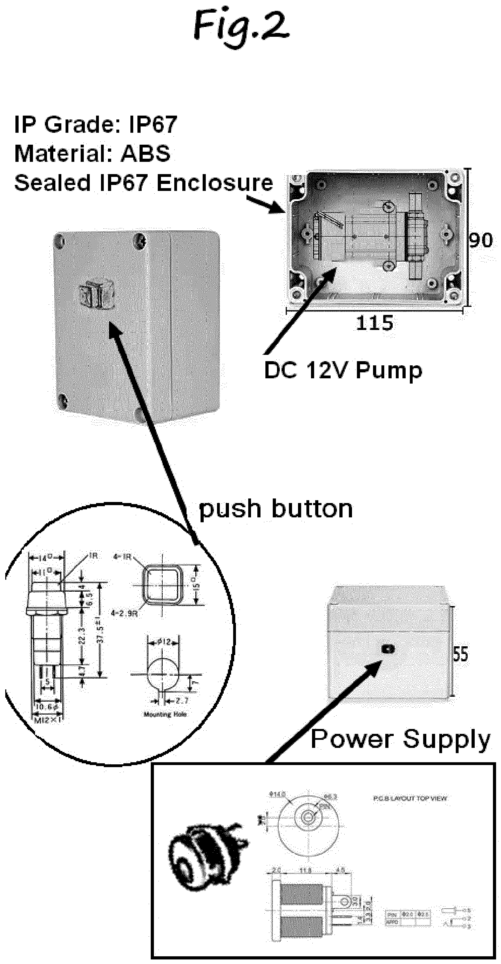

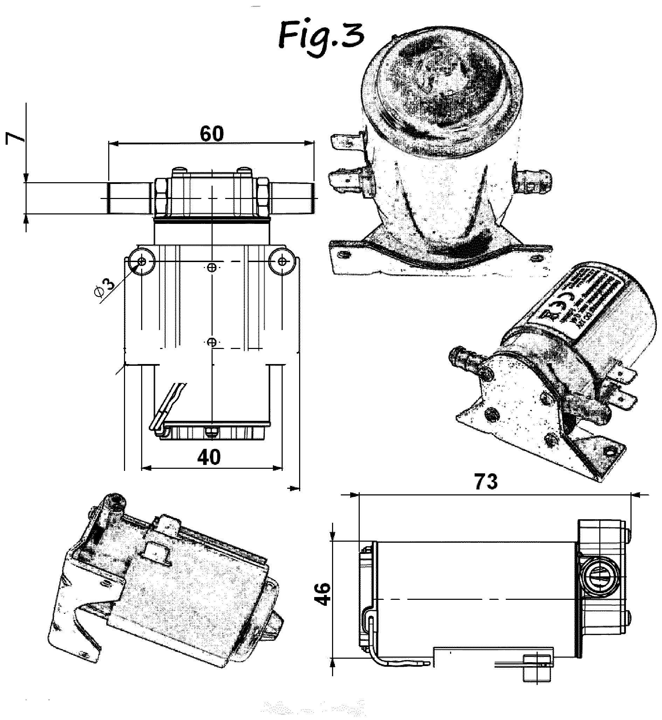

The invention will be explained in more detail below on the basis of an example of the construction according to the invention shown in the figures. The figures are not intended to limit the scope of the invention, but only to illustrate it. In Fig. 1 we see a schematic diagram of the structure of the utility model and shows the entire device and its connections and possibilities in practice. 1 concerns the plastic splash-proof base/housing; 2 concerns the push-button switch; 3 The low-voltage connector type Bus inside Ø 6.3mm; 4 The input unit; 5 The output unit; 6 The power supply adapter; 7 silicone flush line/connection hoses for catheter(s) 8; and or 9 a urine bag; 10 a sink as an example. Fig. 2 shows an open top view of the plastic housing with schematic showing the mounted pump and the sealing rubber for the housing cover. On a 3d front/side view, an example of the push button switch is shown. And a side view front, the socket for adapter (power supply). Fig. 3 shows an example of the pump to be mounted with dimensions. Fig 4 shows two views and dimensions of the housing.

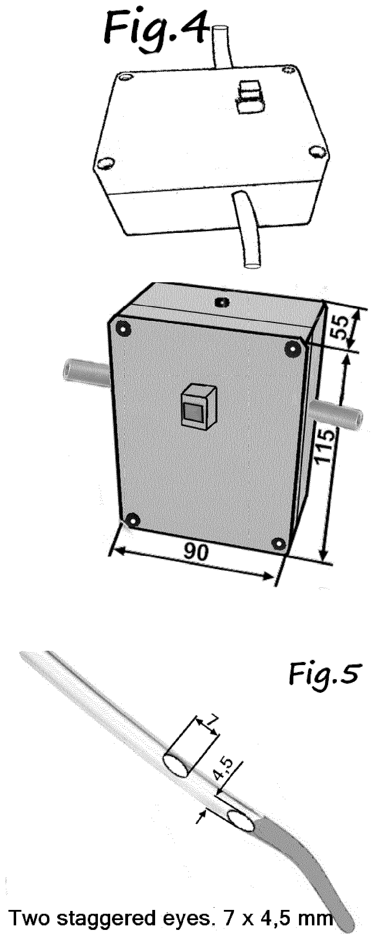

Fig. 5 shows a portion of the Rusch 221800200 Siliconized Tiemann Intermittent Catheter, 20 Ch with the lateral eyes.

The present invention can be explained in detail by means of the following embodiments. The purpose of making the present invention public is to protect all technical improvements within the scope of the present invention. In the description of the present invention and embodiment, it must be understood that if there are terms such as "above", "below", "front", "behind", "left", "right", etc. that indicate directions or positional relationships, they only correspond to the drawings of the present invention. For the convenience of describing the present invention, and do not indicate or imply that the device or element referenced must have a specific orientation or dimension. This also applies to the switch 2, pump and/or power connection 3 used in the description and/or drawings as well as the connection system of the inlet unit 4 and exhaust unit 5.

### CONCLUSIONS

Self-priming electric bladder irrigation device, for connecting a flushing hose to a catheter and a drain flushing hose to a catheter bag or other reservoir. The pump turns on and starts sucking when pressed and held. As soon as it is released, the pump stops sucking. A lot of grit, stones and impurities in the bladder can be effectively removed/drained. The pump will not suck the bladder wall vacuum because of the big double lateral eyes. The patient, helper or nurse can always operate the switch in such a way that the bladder is emptied as well as possible. The device is intended to ensure smooth urine drainage and prevent infection in patients with long-term, or permanent, impaired bladder function. This can be done with a partially filled bladder of at least 100ml of urine and/or by first injecting/squeezing forcefully a bag of bladder irrigation fluid into the bladder trough the catheter. You can also use a 2-way or 3-way catheter to permanently connect a bag of bladder irrigation fluid, or at home if necessary by connecting tap water with a capacity of up to 25 ml/second to the tap and have the device sucking at the same time. In this way, the bladder is flushed even more effectively of impurities. The pump has a capacity of up to 25 ml/second, in order to be able to effectively suck up all stone dust through the large lateral eyes. The device gives a huge boost to bladder management, which gives the possibility to preventively remove bladder stones, prevent clumping of stones, and therefore no longer provide a source for infections.

## Claims

1. Bladder irrigation device, comprising a self-priming electric pump and designed for connecting a flushing hose to a catheter and a drain flushing hose to a catheter bag or other reservoir.

2. Bladder irrigation device according to claim 1, comprising a switch for the ability to turn the pump on and off in such a way that the pump turns on and sucks when pressed and held, and turns off when released.

3. Bladder irrigation device, according to one or more of the foregoing conclusions, optionally designed to be able to be connected by means of a two- or three-way catheter, a bag of bladder irrigation fluid or a tap water connection in order to simultaneously suck in the bladder irrigation fluid or rinse water.
